# EUROPEAN PATENT SPECIFICATION

(11) **EP 2 880 150 B1**
(45) Date of publication and mention of the grant of the patent: **12.10.2016**
(21) Application number: 13745347.8
(22) Date of filing: 01.08.2013
(51) Int. Cl.: C12N 1/20, C12Q 1/04

(54) **TRANSPORT MEDIUM FOR ISOLATION AND IDENTIFICATION OF HELICOBACTER PYLORI**
TRANSPORTMEDIUM ZUR ISOLIERUNG UND IDENTIFIZIERUNG VON HELICOBACTER PYLORI
MILIEU DE TRANSPORT POUR L'ISOLEMENT ET L'IDENTIFICATION D'HELICOBACTER PYLORI

(30) Priority: 02.08.2012 IT RM20120379
(43) Date of publication of application: 10.06.2015
(73) Proprietor: Università Degli Studi "G. D'Annunzio" Chieti-Pescara, 66013 Chieti (CH) (IT)
(72) Inventor: CELLINI, Luigina, I-65015 Montesilvano (IT); DI CAMPLI, Emanuela, 66026 Ortona (CH) (IT); DI BARTOLOMEO, Soraya, I-65124 Pescara (IT)
(74) Representative: Statti, Francesco
(86) International application number: PCT/EP2013/002292
(87) International publication number: WO 2014/019696

(56) References cited:
- DE-C1- 4 227 682
- ANONYMOUS: "BD Helicobacter Agar, Modified", BD (Becton, Dickinson and Company) , June 2003 (2003-06), pages 1-3, XP002692549, Retrieved from the Internet: URL:http://www.bd.com/europe/regulatory/As sets/IFU/HB/CE/PA/PA-254430.pdf [retrieved on 2013-02-21]
- BLANCHARD THOMAS G ET AL: "Laboratory maintenance of Helicobacter species.(Unit8B.1)", CURRENT PROTOCOLS IN MICROBIOLOGY, vol. Chapter 8, February 2012 (2012-02), pages 8B.1.1-8B.1.19., XP002692550, ISSN: 1934-8533, DOI: 10.1002/9780471729259.mc08b01s24
- OWEN R J: "1 Bacteriology of Helicobacter pylori", BAILLIERES CLINICAL GASTROENTEROLOGY, vol. 9, no. 3, 1 September 1995 (1995-09-01), pages 415-446, XP004757677, BAILLIERES TINDALL, LONDON, GB ISSN: 0950-3528, DOI: 10.1016/0950-3528(95)90041-1
- NDIP RN ET AL: "Culturing Helicobacter pylori from Clinical Specimens: Review of Microbiologic Methods", JOURNAL OF PEDIATRIC GASTROENTEROLOGY AND NUTRITION, vol. 36, no. 5, 1 May 2003 (2003-05-01), pages 616-622, XP009167301, LIPPINCOTT WILLIAMS WILKINS, INC, US ISSN: 0277-2116
- Glenda Lakey: "Cary-Blair Transport Medium", Dalynn Biologicals , February 2005 (2005-02), XP002692551, Retrieved from the Internet: URL:http://www.dalynn.com/docs/TC35-10.pdf [retrieved on 2013-02-21]
- VEENENDAAL R A ET AL: "Effect of transport medium and transportation time on culture of Helicobacter pylori from gastric biopsy specimens", JOURNAL OF CLINICAL PATHOLOGY (LONDON), vol. 46, no. 6, 1993, pages 561-563, XP002692552, ISSN: 0021-9746
- VEGA A E ET AL: "Evaluation of a serum-free transport medium supplemented with cyanobacterial extract, for the optimal survival offrom biopsy samples and strains", EUROPEAN JOURNAL OF CLINICAL MICROBIOLOGY & INFECTIOUS DISEASES, SPRINGER, BERLIN, DE, vol. 31, no. 2, 11 May 2011 (2011-05-11), pages 135-139, XP019997861, ISSN: 1435-4373, DOI: 10.1007/S10096-011-1285-Z
- ANONYMOUS: "Brain-Heart Infusion Broth (7116)", Acumedia , 3 November 2010 (2010-11-03), XP002692553, Retrieved from the Internet: URL:http://www.neogen.com/Acumedia/pdf/Pro dInfo/7116_PI.pdf [retrieved on 2013-02-21]
- 'STUART TRANSPORT MEDIUM' REMEL, [Online] 11 January 2010, XP055265600 Retrieved from the Internet: <URL:https://tools.thermofisher.com/content /sfs/manuals/IFU64620.pdf> [retrieved on 2016-04-14]

## Description

### Field of the Invention

The present invention relates to a transport medium for isolation and identification of Helicobacter pylori.

More in detail, the invention relates to a transport medium of the said type, in particular intended for transporting gastric biopsies for culturing and recovering Helicobacter pylori, which is in a semi-solid state and provides optimal conditions for maintaining the viability of the micro-organism.

### Background of the Invention

As is well known, transport media are used for the temporary preservation of specimens during transport to the analysis laboratory for a culture test, and their function is to preserve the viability of all the micro-organisms present, and keep their concentration constant. Generally, transport media for micro-organisms consist of buffered saline solutions, and more particularly, to prevent bacterial replication, carbon, nitrogen and growth agents must not be present in them.

As regards Helicobacter pylori, it has not been possible up to now to develop a transport medium consisting only of buffers and salt. Since it is an easily "stressed" micro-organism, in the presence of a medium consisting only of buffers and salt, Helicobacter pylori responds to the unfavourable environment by acquiring the Viable But Non-Culturable (VBNC) state that does not allow subsequent isolation on culture media.

Currently, transport media used for isolation and identification of Helicobacter pylori have in their composition a certain quantity of antibiotics whose function is to prevent microbial contamination of the medium.

It is also known that Helicobacter pylori is a micro-organism that is difficult to isolate and has three different morphological appearances: i) spiral in vivo when the germ colonizes the gastric mucosa; ii) bacillary in vitro when the germ grows on culture media; iii) coccoid in sub-optimal conditions of growth (lack of nutrients, presence of sub-inhibitory concentrations of antibiotic).

The presence in the transport medium of chemoantibiotics, even those ineffective against Helicobacter pylori, is therefore not desirable, because it can be "stressing" and therefore induce in the microbial population the coccoid morphotype that partly loses its ability to grow on solidifiable culture media (VBNC state).

Further limitations of transport media according to the known art: the poor ability of these media to preserve the specimen over time (also ensuring compliance with the optimal preservation temperature, which is 4°C) and the difficulty of isolating Helicobacter pylori from the medium.

In the current state of the art, as regards transport media for isolation and identification of Helicobacter pylori, the following publications exist:
1. "BD Helicobacter Agar, Modified" that discloses a selective and solidifiable medium, onto which the biopsies are plated, and incubated at 37°C under microaerophilic conditions, with the aim of obtaining microbial growth. A selective medium is a medium that favours the growth only of particular bacterial species thanks to the presence of factors that inhibit the development of other species. These factors are called inhibitory substances and can be antibiotics, dyes or salts; in fact, the components present in the selective medium described in this article also include antibiotics and blood.
2. "Blanchard TG and Nedrud JG. Laboratory Maintenance of Helicobacter Species. Current Protocols in Microbiology 8B.1.1-8B.1.19, February 2012" that discloses a transport medium for biopsies consisting of Brucella broth supplemented with 10% Foetal Bovine Serum (FBS). The medium described in this article is a liquid medium and contains FBS that, as documented in the literature, can show variations from batch to batch, with consequent difference in bacterial growth. According to this article, biopsies should be transferred, in a liquid medium containing FBS, to the Microbiology laboratory within 2-3 hours of being taken.
3. "Owen RJ. Bacteriology of Helicobacter pylori. Bailliere's Clinical Gastroenterology. 9, 1995", in the "Transport of biopsies" section, discloses a transport medium, Portagerm pylori, that has antibiotics among its ingredients to prevent pollution of the biopsy. This characteristic enhances the acquisition of the Viable But Non-Culturable (VBNC) state by Helicobacter pylori with the difficulty of recovering the bacterium, if present, and with the risk of false negative results; all this prolongs the isolation time and therefore the time taken to get the results back to the patient.
4. "Ndip RN, MacKay WG, Farthing MJG, Weaver LT. Culturing Helicobacter pylori from Clinical Specimens: Review of Microbiological Methods. Journal of Pediatric Gastroenterology and Nutrition 36:616-622; May 2003" describes several methods of transporting biopsy specimens, all to be processed within 2 to 3 hours of being taken.

### Disclosure of the Invention

It is an object of the present invention to make a semi-solid transport medium for isolation and identification of Helicobacter pylori that will allow the limitations of solutions according to known technology to be overcome and to obtain the previously described technical results.

It is a further object of the invention that the said semi-solid transport medium can be made with basically reasonable costs, as regards both production costs and preservation costs.

It is still another object of the invention to make a semi-solid transport medium for isolation and identification of Helicobacter pylori that is basically easy to make, safe and reliable.

### Detailed description of Invention

A first specific subject of the present invention is therefore a semi-solid transport medium for isolation and identification of Helicobacter pylori, which comprises, for one litre of medium: 6.5 to 7.2 g granulated agar, 9.5 to 10.5 g enzymatic digest of casein, 9 to 10 g enzymatic digest of animal tissue, 1.8 to 2.2 g yeast extract, 0.8 to 1.2 g glucose, 5.3 to 5.7 g sodium chloride, as well as 9 to 10.2 ml enrichment, the rest being made up of distilled water, since a pH of between 6.8 and 7.2 must be ensured.

Preferably, according to the invention, the said enrichment contains (all components expressed in g/l): Vitamin B12 0.01, L-Glutamine 9.5 to 10.5, Adenine 0.9 to 1.1, Guanine Hydrochloride 0.03, p-Aminobenzoic Acid 0.013, Nicotinamide Adenine Dinucleotide 0.23 to 0.27, Thiamine Pyrophosphate 0.1, Ferric Nitrate 0.02, Thiamine Hydrochloride 0.003, L-Cysteine Hydrochloride 24.9 to 26.9, L-Cystine 1 to 1.2, Dextrose 90 to 100. Even more preferably, according to the invention, the said transport medium comprises, for one litre of medium: 7 g granulated agar, 10 g enzymatic digest of casein, 9.5 g enzymatic digest of animal tissue, 2 g yeast extract, 1 g glucose, 5.5 g sodium chloride, as well as 10 ml enrichment, the rest (961.2 ml) being made up of distilled water, since a pH of 7.0±0.2 must be ensured. Also, a second specific subject of the present invention is a process for making a transport medium as previously defined, comprising the following steps:
- mix all the components, except for the enrichment, in the defined proportions;
- bring the mixture to the boil while stirring;
- sterilize in an autoclave at a temperature of 121°C for 15 minutes or 119°C for 20 minutes;
- cool the mixture to a temperature of between 45°C and 50°C;
- add the enrichment.

In particular, according to the invention, the medium obtained is preserved at a temperature of between 4°C and 8°C.

Preferably, according to the present invention, the said sterilization step is carried out in an autoclave at a temperature of 121°C for 15 minutes, the said cooling step that precedes the addition of the enrichment involves reduction of the temperature to 50°C and the said preservation step is carried out at a temperature of 4°C.

The invention will be described below by way of non-limiting illustration, with particular reference to some illustrative examples.

In particular, the invention refers to a new transport medium for gastric biopsies for recovery of Helicobacter pylori, a micro-organism that is difficult to isolate. It is a sterile, semi-solid, odourless, easy-to-prepare medium whose suitably combined ingredients allow the micro-organism to be recovered over time. The proposed transport medium is without the mixture of antibiotics present in the formulations of Helicobacter pylori transport media according to the known art and in particular the only product present on the Italian market (Portagerm pylori^{®}, by BioMérieux Italy).

As will be made clearer below, these observations suggest the use of this preparation also for a lower cost than the product on the market.

The new transport medium according to the present invention is a semi-solid medium, suitable for transporting difficult bacteria like Helicobacter pylori from the gastroenterology centre to the laboratory. The biopsy specimen is inserted deep into the agar immediately after being taken. The bottles, kept protected from the light, are transported to the laboratory.

The biopsy, recovered in the laboratory, is suitably plated onto suitable selective and non-selective media, for recovery of Helicobacter pylori.

The efficiency of this preparation lies in the possibility of isolating the micro-organism even after a preservation period of 10 days with excellent recovery of the bacterium with no contaminants.

Another interesting aspect is the absence of antibiotics in the composition of the product that does not favour the morphological conversion of Helicobacter pylori from bacillus to coccus, with a faster recovery than the product currently on the market.

This performance allows the antibiogram to be carried out more quickly, thus giving the patient an earlier microbiological result.

The medium is easy to prepare and the ingredients are easy to find.

The essential differences between what is disclosed in the present invention and:
1. the aforementioned publication "BD Helicobacter Agar, Modified", are the following:
   - the medium described therein is a selective, solidifiable medium, whereas the medium of the present invention is a semi-solid medium;
   - the medium described therein is used for isolating the bacterium, whereas the medium of the present invention is for preserving the bacterium;
   - the biopsy of the present invention is stuck into the culture medium and the aim it claims is a suitable transportation of the bacterium, which preserves its viability for up to 10 days, if kept at 4°C.
2. the aforementioned publication "Blanchard TG and Nedrud JG. Laboratory Maintenance of Helicobacter Species. Current Protocols in Microbiology 8B.1.1-8B.1.19, February 2012", are the following:
   - the medium of the said publication is a liquid medium, whereas the medium of the present invention is semi-solid;
   - the FBS present in the said publication is, instead, absent in the medium according to the present invention;
   - the medium according to the present invention allows transportation to the Microbiology laboratory up to 10 days after the biopsy was taken.
3. the aforementioned publication "Owen RJ. Bacteriology of Helicobacter pylori. Bailliere's Clinical Gastroenterology. 9,1995", are the following:
   - the preparation according to the present invention does not have antibiotics among its ingredients. If preserved at 4°C, the biopsy specimen can be taken to the Microbiology laboratory even 10 days after being taken, while allowing the viability and culturability of Helicobacter pylori to be preserved.
4. the aforementioned publication "Ndip RN, MacKay WG, Farthing MJG, Weaver LT. Culturing Helicobacter pylori from Clinical Specimens: Review of Microbiological Methods. Journal of Pediatric Gastroenterology and Nutrition 36:616-622; May 2003": are basically the same differences as those pointed out in comparison to publication 2.

In conclusion, the composition of the medium according to the present invention is the result of experimental work in the laboratory and the ingredients were combined in an optimal manner for preservation of the bacterium and not for its isolation, as instead disclosed in the publications present in the current state of the art.

### Example 1. Composition of the transport medium according to the present invention

The experimental trials described in the description below were carried out using a transport medium for isolation and identification of Helicobacter pylori that had the composition specified in Table 1.

**Table 1.**

| Ingredients for 1 litre | |
|---|---|
| Enrichment (ml)* (Becton-Dickinson 211875, Le Point de Claix, France) | 10 |
| Granulated agar (g) (agar n°1 Bacteriological LP0011B, OXOID Spa, Garbagnate Milanese-MI, Italy) | 7 |
| Enzymatic digest of casein (g) (Tryptone T9410, Sigma-Aldrich, Milan) | 10 |
| Enzymatic digest of animal tissue (g) (peptone from animal tissue, Type I, for microbiology, P7750, Sigma-Aldrich, Milan, Italy) | 9.5 |
| Yeast extract (g) (Yeast Extract power LP021B, OXOID Spa, Garbagnate Milanese-MI, Italy) | 2 |
| Glucose (g) (Glucose 4125012, Biolife Italiana, Milan, Italy) | 1 |
| Sodium chloride (g) (Sodium chloride S7653- 1 Kg, Sigma Aldrich, Milan, Italy) | 5.5 |
| Distilled H₂O (ml) | 961.2 |
| pH | 7.0 ± 0.2 |

| | |
|---|---|
| * IsoVitaleX supplement (all components are expressed in g/l): Vitamin B12 0.01, L-Glutamine 10.0, Adenine 1.0, Guanine Hydrochloride 0.03, p-Aminobenzoic Acid 0.013, Nicotinamide Adenine Dinucleotide 0.25, Thiamine Pyrophosphate 0.1, Ferric Nitrate 0.02, Thiamine Hydrochloride 0.003, L-Cysteine Hydrochloride 25.9, L-Cystine 1.1, Dextrose 100.0. | |

### Example 2. Method of preparing the transport medium according to the present invention

After weighing the ingredients that make up the medium, they were mixed, except for the enrichment, and brought to the boil while stirring and then sterilized in an autoclave at 121°C for 15 minutes. After sterilization, the medium was cooled to 50°C, and then the enrichment was added. The medium was then divided into sterile 1 ml bottles that were easy to open and close with no sharp ends. The cooled medium was then preserved at a temperature of 4°C before use. The medium thus obtained is of a pale colour, with homogeneous consistency and transparent against the light, so as to facilitate detection of the embedded biopsy.

### Example 3. Preservation of the transport medium according to the present invention

The transport medium according to the present invention was preserved at a temperature of 4-6°C, away from the light, in a dry place. In these conditions, the product was then used one and a half years after being prepared, and proved to be still valid.

Any changes in colour, hardening or other obvious signs of deterioration of the agar are an indicator of the fact that preservation has not been carried out correctly and that the medium cannot be used.

### Example 4. Assessment of the efficacy of the transport medium according to the present invention

The efficacy of the transport medium was assessed in vitro and ex vivo.

In vitro, an international reference strain of Helicobacter pylori ATCC 43629 and a clinical strain were inoculated at different concentrations in the transport medium of the present invention, and the recovery of Helicobacter pylori over time was assessed. Table 2 shows the results obtained, in particular the number of CFU (Colony Forming Units) of Helicobacter pylori (reference strain Helicobacter pylori ATCC 43629-Hp and clinical strain Helicobacter pylori 13-Hp2) recovered after preservation at 4°C in the transport medium of the invention over time (T=0, 1, 2, 3, 4, 5 and 7 days).

In particular, in Table 2, the number of CFU per plate is the average of triplicate experiments.
(Table 2 follows)

**Table 2**

| Initial concentration of Helicobacter pylori in the transport medium (CFU) | Recovery of Helicobacter pylori (CFU) after preservation at 4°C over time (days) | | | | | | | | | | | | | | | | | | | |
|---|---|---|---|---|---|---|---|---|---|---|---|---|---|---|---|---|---|---|---|---|
| | 0 | | 1 | | | 2 | | | | 3 | | 4 | | | 5 | | | 7 | | |
| | Hp1 | | Hp2 | Hp1 | Hp2 | | Hp 1 | Hp2 | Hp1 | | Hp2 | | Hp1 | Hp2 | | Hp1 | Hp2 | | Hp1 | Hp2 |
| 10⁵ | >10³ | -* | >10³ | | -* | | >10³ | -* | >10³ | | -* | >10³ | | >10³ | >10³ | | 324 | 150 | | 40 |
| 10⁴ | >10³ | >10³ | >10³ | | 705 | | >10³ | 208 | >10³ | | 155 | 321 | | 98 | 375 | | 8 | 3 | | 0 |
| 10³ | 490 | 106 | 450 | | 52 | | 471 | 44 | 420 | | 6 | 88 | | 1 | 39 | | 0 | 0 | | 0 |
| 10² | 170 | 12 | 165 | | 8 | | 158 | 3 | 33 | | 0 | 7 | | 0 | 5 | | 0 | 0 | | 0 |
| 10¹ | 9 | 2 | 32 | | 2 | | 1 | 0 | 3 | | 0 | 1 | | 0 | 0 | | 0 | 0 | | 0 |

| | | | | | | | | | | | | | | | | | | | | |
|---|---|---|---|---|---|---|---|---|---|---|---|---|---|---|---|---|---|---|---|---|
| * no growth | | | | | | | | | | | | | | | | | | | | |

The results shown in Table 2 point up the efficacy of recovery, over time, of the micro-organism, even at particularly low inoculum concentrations (10 CFU) with its recovery being obtained for up to 4 days.

Ex vivo, 22 biopsies from the gastric antrum and 22 biopsies from the gastric fundus (Table 3) of UBT (Urea Breath Test) positive patients were studied. The suitably subdivided biopsies were placed in bottles of the transport medium of example 1, at a temperature of 4°C, and processed for up to 10 days after they were taken. In all the trials carried out, Helicobacter pylori was recovered. Pollution was found in only one case (specimen 17A/F due to contaminated biopsy forceps).

Compared to Portagerm-pylori, the transport medium of the present invention showed the possibility of allowing recovery and isolation of the bacterium for up to 10 days of preservation of the biopsy specimen at 4°C.

Furthermore, the transport medium of the invention, preserved at a temperature of 4°C for a period of 18 months, maintained the same characteristics, thus allowing good recovery of the micro-organism. Compared to the expiry date indicated in Portagerm-pylori (6 months), the transport medium of the present invention, preserved for 18 months at 4°C, allows an excellent Helicobacter pylori recovery ability with characteristics that are superimposable on the information in Table 3.

In particular, Table 3 shows the characteristics of Helicobacter pylori isolation from gastric biopsies (A-antrum, F-fundus) preserved in the transport medium of example 1 for up to 10 days at a temperature of 4°C (each biopsy specimen was set in 4 fragments, randomly processed).

**Table 3**

| Biopsy specimen | | Testing positive for Helicobacter pylori over time (days) | | | | | | | | |
|---|---|---|---|---|---|---|---|---|---|---|
| Antrum | 1* | 2 | 3 | 4 | 5 | 6 | 7 | 8 | 9 | 10 |
| 7 A 2011 | + | | | | | | | | | |
| 8 A 2011 | + | | | | | | | | | |
| 9 A 2011 | + | | | | | | | | | |
| 10 A 2011 | + | | | | | | | | | |
| 11 A 2011 | + | | | | | | | | | |
| 12 A 2011 | + | | | | | | | | | |
| 13 A 2011 | + | | | | | | | | | |
| 14 A 2011 | + | | | | | | | | | |
| 15 A 2011 | + | | | | | | | | | |
| 16 A 2011 | + | | | | | | | | | |
| 17 A 2011 | - | | | | | | | | | |
| 18 A 2011 | + | | + | | + | | | | + | |
| 19 A 2011 | + | + | | + | | | | | | + |
| 20 A 2011 | + | + | | | | | | + | | + |
| 21 A 2011 | + | | + | | | | | + | | |
| 22 A 2011 | + | | | + | | + | | | | + |
| 23 A 2011 | + | | | | + | | + | + | | |
| 24 A 2011 | + | + | | | | + | | | | + |
| 25 A 2011 | + | | + | | | | | + | | + |
| 1 A 2012 | + | + | | | | + | | | | + |
| 2 A 2012 | + | + | | | | | | | + | + |
| 3 A 2012 | + | | | + | | + | | | | + |
| 4 A 2012 | + | | | + | | | + | | + | |
| 5 A 2012 | + | | + | | + | | | + | | |

| Biopsy specimen | | Testing positive for Helicobacter pylori over time (days) | | | | | | | | |
|---|---|---|---|---|---|---|---|---|---|---|
| Fundus | 1* | 2 | 3 | 4 | 5 | 6 | 7 | 8 | 9 | 10 |
| 7 F 2011 | + | | | | | | | | | |
| 8 F 2011 | + | | | | | | | | | |
| 9 F 2011 | + | | | | | | | | | |
| 10 F 2011 | + | | | | | | | | | |
| 11 F 2011 | + | | | | | | | | | |
| 12 F 2011 | + | | | | | | | | | |
| 13 F 2011 | + | | | | | | | | | |
| 14 F 2011 | + | | | | | | | | | |
| 15 F 2011 | + | | | | | | | | | |
| 16 F 2011 | + | | | | | | | | | |
| 17 F 2011 | - | | | | | | | | | |
| 18 F 2011 | + | | + | | | | + | | + | |
| 19 F 2011 | + | + | | | | + | | | | + |
| 20 F 2011 | + | | | + | | | | + | | + |
| 21 F 2011 | + | | + | | | | | + | | |
| 22 F 2011 | + | + | | + | | | | | | + |
| 23 F 2011 | + | | | | | | + | + | | + |
| 24 F 2011 | + | | | | + | + | | | | + |
| 25 F 2011 | + | | + | | | | | + | | + |
| 1 F 2012 | + | + | | | + | | | | | + |
| 2 F 2012 | + | + | | | | | + | | | + |
| 3 F 2012 | + | | + | + | | | | | | + |
| 4 F 2012 | + | | | + | | + | + | | | |
| 5 F 2012 | + | | + | | + | | | + | | |

| | | | | | | | | | | |
|---|---|---|---|---|---|---|---|---|---|---|
| * 1 st day after being taken | | | | | | | | | | |

In conclusion, the transport medium for isolation and identification of Helicobacter pylori according to the present invention provides optimal conditions for maintaining the viability of the micro-organism.

The simple composition of the transport medium, with the addition of an enrichment supplement favours:
1) a high recovery of Colony Forming Units (CFU);
2) high percentages of isolation from biopsy (>95%);
3) rapid execution of the antibiogram directly from the first isolation;
4) shorter time to result (at least 4 days less) for patients compared to the results obtained with the transport medium according to the known art;
5) low contamination (associated with the composition of the medium and not the presence of antibiotics);
6) low cost.

These advantages highlight the progress achieved with the definition of the transport medium according to the present invention compared to transport media according to the known art. Of considerable importance is respect for the patient, who benefits from a shorter time to result, with therapy being implemented only 7 days after the biopsy was taken; at present, with transport media according to the known art, the result is given about 13 days after the biopsy was taken, with greater difficulty for patients, who are forced to delay eradication therapy. In particular, the problem of Helicobacter pylori eradication regards the resistance of the bacterium to many chemoantibiotics used during therapy; the use of the antibiogram is essential for a targeted choice of the medicines to be used. The transport medium according to the present invention allows direct isolation of Helicobacter pylori even 3 days after the biopsies were plated, with pollutant-free plates, and allows execution of the antibiogram without sub-isolating the strains, with further waiting time for the patient. The success of the rapid recovery of Helicobacter pylori in culture from gastric biopsies is affected by the conditions of transport from the endoscopy room to the laboratory and by correct preservation at a temperature of 4°C.

The present invention has been described by way of non-limiting illustration, according to its preferred embodiments, but it is to be understood that variations and/or modifications may be made by the experts in the field without for this reason departing from its protective scope, as defined by the attached claims.

## Claims

1. Transport medium for gastric biopsies for recovery of Helicobacter pylori, **characterized in that** it is in a semi-solid state and provides conditions for maintaining the viability of the micro-organism, comprising for one litre of medium: 6.5 to 7.2 g granulated agar, 9.5 to 10.5 g enzymatic digest of casein, 9 to 10 g enzymatic digest of animal tissue, 1.8 to 2.2 g yeast extract, 0.8 to 1.2 g glucose, 5.3 to 5-7 g sodium chloride, as well as 9 to 10.2 ml enrichment containing (all components being expressed in g/l): Vitamin B12 0.01, L-Glutamine 9.5 to 10.5, Adenine 0.9 to 1.1, Guanine Hydrochloride 0.03, p-Aminobenzoic Acid 0.013, Nicotinamide Adenine Dinucleotide 0.23 to 0.27, Thiamine Pyrophosphate 0.1, Ferric Nitrate 0.02, Thiamine Hydrochloride 0.003, L-Cysteine Hydrochloride 24.9 to 26.9, L-Cystine 1 to 1.2, Dextrose 90 to 100, the rest being made up of distilled water, since a pH of between 6.8 and 7.2 must be ensured.

2. Transport medium according to claim 1, **characterized in that** it comprises, for one litre of medium: 7 g granulated agar, 10 g enzymatic digest of casein, 9.5 g enzymatic digest of animal tissue, 2 g yeast extract, 1 g glucose, 5.5 g sodium chloride, as well as 10 ml enrichment, the rest (961.2 ml) being made up of distilled water, since a pH of 7.0 ± 0.2 must be ensured.

3. Process of making a transport medium as defined in claims 1-2, **characterized in that** it comprises the following steps:
- mix all the components, except for the enrichment, in the defined proportions;
- bring the mixture to the boil while stirring;
- sterilize in an autoclave at a temperature of 121°C for 15 minutes or 119°C for 20 minutes;
- cool the mixture to a temperature of between 45°C and 50°C;
- add the enrichment.

4. Process according to claim 3, **characterized in that** it further envisages that the medium obtained be preserved at a temperature of between 4°C and 8°C.

5. Process according to either one of claims 3 and 4, **characterized in that** the said sterilization step is carried out in an autoclave at a temperature of 121°C for 15 minutes.

6. Process according to any one of claims 3-5, **characterized in that** the said cooling step that precedes the addition of the enrichment involves reduction of the temperature to 50°C.

7. Process according to any one of claims 3-6, **characterized in that** the said preservation step is carried out at a temperature of 4°C.

## Patentansprüche

1. Transportmedium für Magen-Biopsien zur Isolierung von Helicobacter Pylori, **dadurch gekennzeichnet, dass** es in einem halbfesten Zustand ist und Bedingungen schafft, zum Erhalten der Lebensfähigkeit des Mikroorganismus, für einen Liter des Mediums bestehend aus: 6,5 bis 7,2 g granuliertem Agar-Agar, 9,5 bis 10,5 g enzymatisch verdautem Kasein, 9 bis 10 g enzymatisch verdautem Tiergewebe, 1,8 to 2,2 g Hefeextrakt, 0,8 bis 1,2 g Glukose, 5,3 bis 5,7 g Natriumchlorid, sowie 9 bis 10,2 ml Anreicherung bestehend aus (alle Komponenten werden ausgedrückt in g/l): Vitamin B12 0,01, L-Glutamin 9,5 bis 10,5, Adenin 0,9 bis 1,1, Guaninhydrochlorid 0,03, p-Aminobenzosäure 0,013, Nicotinamidadenin Dinukleotid 0,23 bis 0,27, Thiaminpyrophosphat 0,1, Eisennitrat 0,02, Thiaminhydrochlorid 0,003, L-Cysteinhydrochlorid 24,9 bis 26,9, L-Cystin 1 bis 1,2, Dextrose 90 bis 100, der Rest besteht aus destilliertem Wasser, da ein pH-Wert zwischen 6,8 bis 7,2 gewährleistet werden muss.

2. Transportmedium nach Anspruch 1, **dadurch gekennzeichnet, dass** es für einen Liter Medium umfasst: 7 g granuliertem Agar-Agar, 10 g enzymatisch verdautes Kasein, 9,5 g enzymatisch verdautes Tiergewebe, 2 g Hefeextrakt, 1 g Glukose, 5,5 g Natriumchlorid, sowie 10 ml Anreicherung, der Rest (961,2 ml) besteht aus destilliertem Wasser, da ein pH-Wert zwischen 7,0 ± 0,2 gewährleistet werden muss.

3. Verfahren zur Herstellung eines Transportmediums wie in den Ansprüchen 1 bis 2, **dadurch gekennzeichnet, dass** es die folgenden Schritte umfasst:
- alle Komponenten in den festgelegten Anteilen mischen, außer der Anreicherung;
- die Mischung unter Rühren zum Kochen bringen;
- in einem Autoklav bei einer Temperatur von 121°C für 15 Minuten oder von 119°C für 20 Minuten sterilisieren;
- die Mischung auf eine Temperatur von 45°C bis 50°C abkühlen;
- die Anreicherung hinzufügen.

4. Verfahren nach Anspruch 3, **dadurch gekennzeichnet, dass** es weiter vorsieht, dass das erhaltene Medium bei einer Temperatur zwischen 4°C bis 8°C aufbewahrt wird.

5. Verfahren nach einem der Ansprüche 3 und 4, **dadurch gekennzeichnet, dass** der besagte Sterilisationsschritt in einem Autoklav bei einer Temperatur von 121°C für 15 Minuten durchgeführt wird.

6. Verfahren nach einem der Ansprüche 3 bis 5, **dadurch gekennzeichnet, dass** die besagten Abkühlungsschritte, die dem Zusatz der Anreicherung vorausgehen, eine Senkung der Temperatur auf 50°C vorsieht.

7. Verfahren nach einem der Ansprüche 3 bis 6, **dadurch gekennzeichnet, dass** der besagte Konservierungsschritt bei einer Temperatur von 4°C erfolgt.

## Revendications

1. Milieu de transport destiné aux biopsies gastriques pour l'éradication de Helicobacter pylori, **caractérisé par le fait que** celui-ci est à l'état semi-solide et présente les conditions permettant de maintenir la viabilité du micro-organisme, comprenant pour un litre de milieu: 6,5 à 7,2 g de gélose granulée, 9,5 à 10,5 g de digestat enzymatique de caséine, 9 à 10 g de digestat enzymatique de tissus animaux, 1,8 à 2,2 g d'extrait de levure, 0,8 à 1,2 g de glucose, 5,3 à 5,7 g de chlorure de sodium, ainsi que 9 à 10,2 ml d'enrichissement contenant (tous les composants étant exprimés en g/l): 0,01 g/l de vitamine B12, 9,5 à 10,5 g/l de L-glutamine, 0,9 à 1,1 g/l d'adénine, 0,03 g/l de chlorhydrate de guanine, 0,013 g/l d'acide para-aminobenzoïque, 0,23 à 0,27 g/l de nicotinamide adénine dinucléotide, 0,1 g/l de pyrophosphate de thiamine, 0,02 g/l de nitrate ferrique, 0,003 g/l de chlorhydrate de thiamine, 24,9 à 26,9 g/l de chlorhydrate de L-cystéine, 1 à 1,2 g/l de L-cystine, 90 à 100 g/l de dextrose, le reste étant constitué d'eau déminéralisée, puisqu'un pH compris entre 6,8 et 7,2 doit être garanti.

2. Milieu de transport selon la revendication 1, **caractérisé en ce qu'**il comprend, pour un litre de medium: 7 g de gélose granulée, 10 g de digestat enzymatique de caséine, 9,5 g de digestat enzymatique de tissus animaux, 2 g d'extrait de levure, 1 g de glucose, 5,5 g de chlorure de sodium, ainsi que 10 ml d'enrichissement, le reste (961,2 ml) étant constitué d'eau déminéralisée, puisqu'un pH de 7,0 ± 0,2 doit être garanti.

3. Procédé de production d'un milieu de transport tel que défini dans les revendications 1 et 2, **caractérisé en ce qu'**il comprend les étapes suivantes, consistant à:
- mélanger tous les composants, excepté pour l'enrichissement, dans des proportions déterminées;
- porter le mélange à ébullition sous agitation;
- stériliser le mélange à l'autoclave à une température de 121°C pendant 15 minutes ou de 119°C pendant 20 minutes;
- refroidir le mélange à une température se situant entre 45°C et 50°C;
- ajouter l'enrichissement.

4. Procédé selon la revendication 3, **caractérisé en ce qu'**il prévoit en outre que le milieu obtenu puisse être conservé à une température comprise entre 4°C et 8°C.

5. Procédé selon l'une ou l'autre des revendications 3 et 4, **caractérisé en ce que** l'étape de stérilisation s'effectue en autoclave à une température de 121°C pendant 15 minutes.

6. Procédé selon l'une quelconque des revendications 3 à 5, **caractérisé en ce que** l'étape de refroidissement précédant l'adjonction de l'enrichissement implique une réduction de la température à 50°C.

7. Procédé selon l'une quelconque des revendications 3 à 6, **caractérisé en ce que** l'étape de conservation s'effectue à une température de 4°C.
